# EUROPEAN PATENT APPLICATION

(11) **EP 4 846 861 A1**
(43) Date of publication of application: **02.12.2026**
(21) Application number: 25745390.2
(22) Date of filing: 24.01.2025
(51) Int. Cl.: A23L 33/105, A61K 36/15, A61K 36/9062, A61K 31/192, A61K 31/11, A61P 25/28

(54) **PHARMACEUTICAL COMPOSITION COMPRISING HL-NEUROPION (COMPLEX OF PINUS DENSIFLORA EXTRACT AND ALPINIA OFFICINARUM EXTRACT) AS ACTIVE INGREDIENT FOR IMPROVING COGNITIVE FUNCTION AND TREATING DEMENTIA THROUGH EXCELLENT ANTI-INFLAMMATORY EFFECT, INHIBITION OF NEUROTRANSMITTER DEGRADATION, AND INHIBITION OF AMYLOID BETA AGGREGATION**

(30) Priority: 26.01.2024 KR 20240012539
(71) Applicant: HLscience Co., Ltd, Hwaseong-si, Gyeonggi-do 18329 (KR)
(72) Inventor: LEE, Hae-Yeon, Uiwang-si, Gyeonggi-do 16000 (KR); KIM, Jong-Lae, Uiwang-si, Gyeonggi-do 16009 (KR); LEE, Jong-Wook, Suwon-si, Gyeonggi-do 16356 (KR); IM, Sung Bin, Anyang-si, Gyeonggi-do 14038 (KR); YANG, Jin-Seong, Hwaseong-si, Gyeonggi-do 18310 (KR)
(74) Representative: Høiberg P/S
(86) International application number: PCT/KR2025/001467
(87) International publication number: WO 2025/159568

(57) **Abstract**

The present invention relates to: a health functional food, food product, or therapeutic pharmaceutical composition for preventing or ameliorating dementia and cognitive decline, wherein a complex of a Pinus densiflora extract and an Alpinia officinarum extract is included as an active ingredient; and a method for producing same. The complex provided by the present invention was not only found to have excellent acetylcholinesterase inhibitory activity and nitric oxide production inhibitory activity, but was also found to have an activity of improving cognitive decline induced by beta amyloid administration in an animal model, and can thus be used as a health functional food and pharmaceutical for preventing, ameliorating, or treating dementia and cognitive decline.

## Description

### TECHNICAL FIELD

This application claims priority to and the benefit of Korean Patent Application No. 10-2024-0012539, filed on January 26, 2024, the entire disclosure of which, including the specification and drawings, is incorporated herein by reference. The present invention relates to a pharmaceutical composition for preventing, improving or treating dementia and cognitive decline, and more particularly, to a pharmaceutical composition, a health functional food, or a method for preparing the same, which comprises a complex of pine needle extract and galangal extract as an active ingredient and simultaneously imparts excellent inhibitory effects on nitric oxide production and acetylcholinesterase activity, an anti-inflammatory effect, an inhibitory effect on the degradation of a neurotransmitter, and an inhibitory effect on beta-amyloid aggregation, thereby providing an effect of preventing, improving or treating dementia and cognitive decline.

### BACKGROUND ART

As of 2022, the population aged 65 or older in the Republic of Korea was approximately 9 million, and population aging in Korea is accelerating at the fastest rate in the world due to a decline in the birth rate and an increase in life expectancy. As a result of this rapid population aging, the prevalence of dementia is also rapidly increasing. According to statistics from the National Institute of Dementia, approximately 10.3% of the population aged 65 or older, and approximately 35% or more of the population aged 85 or older, have been diagnosed with dementia, and this increasing trend is expected to continue, with the number of dementia patients projected to reach approximately 1 million by 2025 and approximately 3 million by 2050. As the number of dementia patients increases, the social burden caused by dementia is also increasing; the total annual cost of managing dementia patients in 2021 was approximately KRW 18.7 trillion, accounting for approximately 11% of Korea's gross domestic product (GDP), and the annual management cost tends to increase as the severity of dementia increases. In addition, because dementia is often accompanied by behavioral and psychological symptoms, the economic and social costs of dementia already carry a significance beyond what the statistics show, given the additional burden of nursing care and informal care by family members, on top of medication and medical costs.

Although the causes of degenerative neurological diseases such as dementia and the resulting cognitive decline have not yet been precisely identified, various studies suggest that plaque formation due to beta-amyloid aggregation, deposition due to abnormal tau protein, and tissue damage due to oxidative stress or neuroinflammation are receiving attention as major causes. Among these, neuroinflammation is generally a mechanism that protects the brain from direct or indirect damage caused by infection, toxic substances, and the like; however, when an imbalance in inflammation-related signals in the body occurs due to various additional factors, it shifts into a chronic inflammatory response, which adversely affects nerve cells and tissue and causes direct neurodegeneration. In addition, this inflammatory response further promotes the formation of beta-amyloid plaques and tau protein aggregates, and the resulting abnormal proteins in turn induce an inflammatory response, leading to a vicious cycle of disease progression. In this regard, research targeting neuroinflammation is being actively conducted.

Meanwhile, dementia therapeutics currently in use are aimed at improving the cognitive abilities of dementia patients, and in the case of patients with mild to moderate dementia, the focus is on inhibiting the activity of the cholinesterase enzyme, which hydrolyzes acetylcholine-a neurotransmitter that plays an important role in memory and cognitive function in the brain-so as to maintain normal levels of acetylcholine. Representative drugs used for this purpose include donepezil, rivastigmine, and galantamine; however, side effects such as loss of appetite, nausea, vomiting, diarrhea, headache, and weight loss have been reported, which may adversely affect the health of elderly patients. Moreover, these drugs have a clear drawback in that abrupt discontinuation of administration due to such side effects can cause a severe deterioration in cognitive function. Accordingly, there is growing demand for the development of alternative therapies based on combinations of natural ingredients that are expected to minimize side effects and harm to the human body while providing reliable preventive and ameliorative effects.

Throughout this specification, a number of publications are referenced and citations thereof are indicated. The disclosures of the cited publications are incorporated herein by reference in their entirety, so as to more clearly explain the level of the technical field to which the present invention pertains and the subject matter of the present invention.

### DISCLOSURE

### Technical Problem

An object of the present invention is to provide a health functional food and pharmaceutical composition, and a method for preparing the same, capable of exhibiting an effect of preventing or improving cognitive decline and treating dementia, by simultaneously imparting an anti-inflammatory effect, an acetylcholinesterase inhibitory effect, and an effect of improving beta-amyloid-induced cognitive decline, through a natural ingredient or a complex thereof.

### Technical Solution

In order to solve the above-described problem, the present invention provides a health functional food and pharmaceutical composition for preventing, improving or treating dementia and cognitive decline, comprising a complex of pine needle extract and galangal extract as an active ingredient, and a method for preparing the same. In the present invention, the complex is not limited thereto, but is capable of inhibiting acetylcholinesterase activity and nitric oxide production, and exhibits excellent cognitive function-improving efficacy in a beta-amyloid-induced animal model, and therefore can be used as a composition for preventing, improving or treating dementia and cognitive decline. More specifically, it has been confirmed that the complex has an anti-inflammatory activity of inhibiting the production of nitric oxide, which is generated by an inflammatory response and causes damage to cells and tissue, while simultaneously activating neurotransmission through inhibition of the acetylcholinesterase enzyme, which is a representative cause of cognitive decline, as well as an activity of improving cognitive decline induced by administration of beta-amyloid in an animal model. Accordingly, the complex of pine needle extract and galangal extract of the present invention can be used as an active ingredient of a composition for preventing, improving or treating dementia and cognitive decline.

The pine needle used in the present invention is a needle of *Pinus densiflora*, which is an evergreen gymnosperm belonging to the family Pinaceae, order Pinales, and grows wild throughout the Republic of Korea, as well as throughout most of Northeast Asia, including eastern Russia, northeastern China, and Japan. *Pinus densiflora* grows wild on dry mountain ridges with abundant sunlight, and has a height of approximately 35 m and a diameter of approximately 1.8 m. Its needles grow in clusters of two and have a needle-like shape with a pointed tip, with a length of approximately 8 to 9 cm and a width of 1.5 mm. The needles, pollen, and bark of *Pinus densiflora* are used for food or medicinal purposes, and among these, the needles have long been used, for example, by being dried and ground into a powder for use in foods such as tea and rice cakes, or as recorded for medicinal use in old texts such as Donguibogam and Bencao Gangmu (Compendium of Materia Medica). Pine needles are known to contain essential oil components of the terpene series, such as alpha-pinene and beta-pinene, polyphenols such as chlorophyll, lutein, and beta-carotene, as well as vitamin C, vitamin K, flavonoids, and organic acids, and various physiological activities of these bioactive components have been reported, including an effect of lowering serum cholesterol, an anti-diabetic effect, a lipid-lowering effect, an antioxidant effect, and an anticancer and antibacterial effect.

The galangal used in the present invention is the rhizome of *Alpinia officinarum*. The name "galangal" derives from "Gao Liang Jiang," as it was called in China, which began to be referred to as "galangal" after being introduced by the 17th-century Italian botanist Prospero Alpini. In Korea, it is also known as goryanggang, Asian ginger, singang, or yanggang. *Alpinia officinarum* is a plant belonging to the family Zingiberaceae, order Zingiberales, originating from China and cultivated mainly in Southeast Asia. It grows to a height of approximately 1.5 to 2 m and has long leaves and reddish-white flowers. The galangal rhizome has a sweet, pungent taste and aroma, and has been used throughout Asia as an ingredient in curries and perfumes, and has also been widely used in Europe. Galangal is known to contain essential oil components such as 1,8-cineole, methyl cinnamate, and alpha-cadinene, flavonoids such as galangin, kaempferide, and alpinin, and galangol, and various physiological activities of these bioactive components have been reported, including inhibition of pancreatic lipase activity, inhibition of fatty acid synthesis, an antioxidant effect, an effect of protecting cells and DNA from damage caused by reactive oxygen species, and an anti-inflammatory effect such as inhibition of cyclooxygenase-2 and inhibition of prostaglandin biosynthesis.

Accordingly, the present inventors completed the present invention upon discovering that a complex of pine needle extract and galangal extract simultaneously exhibits excellent inhibitory effects on nitric oxide production and acetylcholinesterase activity, thereby simultaneously imparting an anti-inflammatory effect and an inhibitory effect on the degradation of a neurotransmitter, and is thus capable of exhibiting an effect of preventing, improving and treating dementia and cognitive decline.

In one embodiment of the present invention, the pine needle extract is characterized in that it is obtained by extracting pine needles with water, a lower alcohol having 1 to 4 carbon atoms, or a mixed solvent thereof, followed by concentration.

In another embodiment of the present invention, the galangal extract is characterized in that it is obtained by extracting galangal with water, a lower alcohol having 1 to 4 carbon atoms, or a mixed solvent thereof, followed by concentration.

In still another embodiment of the present invention, the pine needle extract or the galangal extract is characterized in that it is a concentrate or a dry powder.

In still another embodiment of the present invention, the complex of the pine needle extract and the galangal extract is characterized in that the galangal extract is mixed with the pine needle extract in a weight ratio of 1:1 to 1:8 (pine needle extract:galangal extract). More specifically, the weight ratio of the pine needle extract to the galangal extract may be 1:2 to 1:8, or 1:4 to 1:8. In yet another embodiment, the weight ratio of the pine needle extract to the galangal extract may be 1:8±1, 1:8±0.5, or 1:8±0.1. By including the pine needle extract and the galangal extract in the above-described weight ratio, the complex of the present invention can exhibit a synergistic effect resulting from the combination, as compared to each extract alone, and can further exhibit an even more remarkable synergistic effect as compared to a case in which the two extracts are included in a different weight ratio.

In still another embodiment of the present invention, the complex may consist of the pine needle extract and the galangal extract, and the weight ratio of the extracts in the complex is as described above.

In still another embodiment of the present invention, the composition of the present invention may not contain any natural extract component, other than the pine needle extract and the galangal extract, as an active ingredient.

In still another embodiment of the present invention, the complex of the pine needle extract and the galangal extract is characterized by an acetylcholinesterase inhibitory activity, an effect of improving cognitive function with respect to beta-amyloid-induced cognitive decline, and a nitric oxide production-inhibitory activity.

In still another embodiment of the present invention, the composition is characterized in that it is a health functional food composition or a pharmaceutical composition.

In addition, the present invention provides a health functional food product and a pharmaceutical product comprising the composition.

Preferably, all of the components described in the present invention do not exceed the maximum amounts of use prescribed by the relevant laws and regulations of Korea, China, the United States, Europe, Japan, and the like (for example, the Korean Food Code, the Korean Food Additives Code, and the Korean Health Functional Food Code). That is, preferably, the food, health functional food, or pharmaceutical composition according to the present invention contains the components according to the present invention within the content limits permitted by the relevant laws and regulations of each country.

### Advantageous Effects

It has been confirmed that the complex provided by the present invention exhibits an anti-inflammatory activity of inhibiting the production of nitric oxide, which is generated by an inflammatory response and causes damage to cells and tissue, while simultaneously activating neurotransmission through inhibition of the acetylcholinesterase enzyme, a representative cause of dementia and cognitive decline, as well as an activity of improving cognitive decline in an animal model in which dementia was induced by administration of beta-amyloid, and since this effect is excellent, the complex can be utilized as a health functional food and pharmaceutical composition for preventing, improving or treating dementia and cognitive decline.

### DESCRIPTION OF DRAWINGS

FIG. 1 is a diagram showing the concentration-dependent anti-inflammatory effect of Example 7.
FIG. 2 is a diagram showing the cognitive function-improving effect of oral administration of Example 4, Example 6, and Example 7 in mice in which an Alzheimer's disease-like state was induced by beta-amyloid.

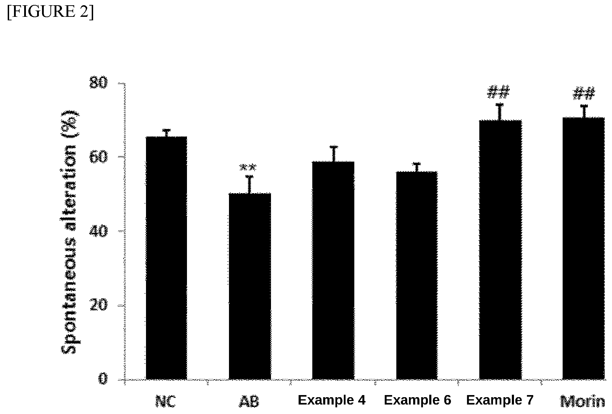

The experimental groups and the graph of FIG. 2 are described below.
NC: control group
AB: beta-amyloid-administered group (induction group)
Example 4: induction group + Example 4 (pine needle extract:Moringa extract = 1:1) 100 mg/kg-administered group
Example 6: induction group + Example 6 (pine needle extract:Moringa extract = 1:4) 100 mg/kg-administered group
Example 7: induction group + Example 7 (pine needle extract:Moringa extract = 1:8) 100 mg/kg-administered group
Morin: induction group + Morin 10 mg/kg-administered group
**: p<0.01 vs. NC by unpaired t-test
##: p<0.01 vs. AB by unpaired t-test

### MODE FOR DISCLOSURE

The present invention provides a composition for preventing, improving or treating dementia and/or cognitive decline, comprising a complex of pine needle extract and galangal extract (HL-BCRS) as an active ingredient, and more specifically, relates to a health functional food and pharmaceutical composition for preventing, improving or treating dementia and cognitive decline.

### [Extract]

In the present invention, the term 'extract' includes an extract solution obtained by an extraction process using water or another solvent, a diluted solution or a concentrated solution of the extract solution, a dried product obtained by drying the extract solution, a crude purified product or a purified product of the extract solution, or a mixture thereof, and the extract solution itself, as well as an extract in any formulation that can be formed using the extract solution.

To the extent that the object of the present invention is not impaired, the extract according to the present invention may include, in addition to the specific parts of the above-described materials, leaves, stems, roots, bark, flowers, fruits, seeds, sap, the whole plant, and the like.

For the preparation of the extract according to the present invention, any suitable method known in the art may be used in the processes before and after extraction, such as pretreatment of the raw material, extraction of the raw material, and processing of the extract. For example, the entirety or an arbitrary portion of the raw material to be used may be pulverized, and then an extraction solvent may be applied thereto to obtain a liquid solvent extract. During the extraction process, the extraction efficiency may be increased by heating, and in this case, an extraction method that minimizes solvent loss may be used by performing a reflux cooling process together, so as to minimize the solvent loss caused by heating. In addition, the raw material may be dried and pulverized prior to the extraction process, and the solvent extract obtained after extraction may be prepared in the form of a filtrate, a concentrate, or a powder, through an additional process such as filtration, vacuum distillation, freeze drying, or spray drying.

The type of extraction solvent used in the extraction process is not particularly limited, and any solvent commonly used in the art may be used. For example, water; a C1 to C4 lower alcohol such as methanol, ethanol, propyl alcohol, or butyl alcohol; a hydrocarbon-based solvent such as ethyl acetate, acetone, hexane, or dichloromethane; or a mixture thereof may be used, and preferably, water or a lower alcohol may be used alone, or two or more thereof may be used in combination. A solvent extract may be prepared by performing extraction one time, or repeating the extraction two or more times, using the above-described solvent, and the solvent extract may be dried to prepare a dry extract.

In the extraction process, the amount of the extraction solvent used may vary depending on the type of solvent used, but, for example, may be used in an amount of 5 to 30 times, more preferably 10 to 20 times, the dry weight of the target raw material. Conditions such as the extraction temperature, time, and pressure are not limited, but extraction may be performed at 60 to 120°C, for 1 to 10 hours, and at 0.1 to 0.3 MPa.

In addition, various extraction processes commonly used in the art, such as leaching, microwave-assisted extraction, ultrasonic extraction, and supercritical fluid extraction, may be selectively used, and these methods may be performed simultaneously or sequentially in combination.

The pine needle extract according to the present invention may contain 0.05 to 0.35 mg/g of protocatechuic acid, and since the reason that protocatechuic acid can be obtained at such a concentration is presumed to be attributable to differences in the origin of the raw material, the type of the part used, the above-described preparation process, and differences in process parameters, the present invention is not limited thereto.

The galangal extract according to the present invention may contain 0.04 to 0.20 mg/g of 4-hydroxybenzaldehyde, and since the reason that 4-hydroxybenzaldehyde can be obtained at such a concentration is presumed to be attributable to differences in the origin of the raw material, the type of the part used, the above-described preparation process, and differences in process parameters, the present invention is not limited thereto.

### [Composition]

The present invention provides a composition usable for preventing, improving or treating dementia and cognitive decline. More specifically, the present invention provides a composition usable for preventing, improving or treating dementia and cognitive decline, comprising a complex of pine needle extract and galangal extract as an active ingredient.

In the present invention, the expression 'comprised as an active ingredient' means that the corresponding component is added to the composition according to the present invention in an amount sufficient to exhibit an effect of preventing, improving or treating dementia and cognitive decline, and this expression encompasses the meaning that the composition may be formulated into various forms through the addition of an auxiliary component other than the active ingredient, for example, for delivery to a target tissue or for stabilization of the composition.

In the composition according to the present invention, the pine needle extract and the galangal extract may have a ratio (weight ratio) of 1:1 to 1:8. Depending on the intended effect of the composition or the form of the product into which the composition is incorporated, the extract, the concentrate, and the complex may be included in an amount ranging from about 0.0001% to 99% or more, based on the final form, which may be calculated based on weight relative to the total weight of the composition, or volume relative to the total volume of the composition.

The composition according to the present invention may be prepared by mixing the pine needle extract and the galangal extract prepared by the above-described method, and may otherwise be prepared by further adding various processes and additives, for example, for the purpose of improving the storability, distributability, or stability of the composition, to the extent that the object of the present invention is not impaired.

The composition according to the present invention may be commercialized as a health functional food composition, a pharmaceutical composition, or the like.

The present invention provides a pharmaceutical composition comprising one of the above-described compositions. In addition, the present invention provides a method of preventing, improving or treating dementia and cognitive decline, comprising administering one of the above-described compositions.

The pharmaceutical composition according to the present invention may comprise a pharmaceutically effective amount of the pine needle extract and the galangal extract alone, or may further comprise a carrier, an excipient, or a diluent commonly used in the art.

The above-described 'pharmaceutically effective amount' refers to an amount that exhibits a response greater than that of a negative control group, and preferably refers to an amount sufficient to exhibit a preventive and therapeutic effect on dementia and cognitive decline.

The above-described 'pharmaceutically acceptable' refers to a physiologically acceptable, non-toxic composition that does not inhibit the action of the active ingredient upon administration to the human body and does not typically cause an allergic reaction, such as a gastrointestinal disorder or dizziness, or a similar side effect.

The carrier, the excipient, and the diluent may be a raw material commonly used in the art, to the extent that the object of the present invention is not impaired, and may further comprise various auxiliary materials, such as a filler, an anti-caking agent, a lubricant, a wetting agent, a flavoring agent, an emulsifier, and a preservative.

In addition, the composition may be formulated using a method commonly known in the art, in order to smoothly achieve the intended purpose using the active ingredient. This may include a powder, granules, a tablet, a syrup, an emulsion, an aerosol, a sterile injectable solution, and a soft or hard capsule.

The pharmaceutical composition of the present invention may be administered through an oral or parenteral route, but the present invention is not limited thereto. In the case of including oral administration, the composition may be prepared by a conventional method together with a pharmaceutically acceptable excipient, and may be formulated into a form suitable for sustained release, controlled release, extended release, or the like, in order to smoothly achieve the intended purpose. In the case of parenteral administration, this may include various routes such as transdermal, intranasal, intraperitoneal, subcutaneous, intramuscular, and intravenous routes, and the composition may likewise be prepared by a conventional method together with a pharmaceutically acceptable excipient.

The preferred dosage of the pharmaceutical composition of the present invention may vary depending on the condition of the patient, the severity of the disease, the form of the drug, and the route and duration of administration, and accordingly, the concentration and dosage of the composition may be appropriately selected by one of ordinary skill in the art.

The pharmaceutical composition of the present invention may be used alone, or in combination with a method of preventing and treating dementia and cognitive decline that is commonly used in the art.

The present invention provides a health functional food composition comprising one of the above-described compositions. The present invention provides a method of preventing, improving or treating dementia and cognitive decline, comprising ingesting one of the above-described compositions.

The health functional food composition according to the present invention may comprise a food-pharmacologically effective amount of the pine needle extract and the galangal extract alone, or may further comprise a carrier, an excipient, or a diluent acceptable in the art.

The health functional food composition according to the present invention includes any processed form, such as a food, a functional food, a nutritional supplement, a health food, and a food additive. The food composition may be prepared in various forms according to a conventional method known in the art, and the form, type, and the like of the finished product are not limited.

The food composition according to the present invention may be added to a food as is, or may be used in combination with another food or food ingredient, and may be appropriately used according to a conventional method. The amount of the active ingredient to be mixed may be appropriately determined according to the intended use (prevention, amelioration, or treatment).

The food composition according to the present invention has no particular limitation on components other than the above-indicated blending ratio of the pine needle extract and the galangal extract, which are essential components, and like conventional products, may contain various flavoring agents, natural carbohydrates, nutrients, vitamins, minerals, flavoring agents, coloring agents and enhancers, other food additives commonly used in the art for various purposes, natural fruits, and fruit pulp for the preparation of vegetable beverages.

### [Use for Preventing, Improving or Treating Dementia and Cognitive Decline, and Treatment Method]

The composition according to the present invention is effective in preventing, improving or treating dementia and/or cognitive decline.

Dementia is an acquired and irreversible intellectual impairment caused by an organic disorder of the brain. It refers to a loss of a person's mental (intellectual) ability and ability to engage in social activities, and is a condition in which intelligence that was once normally developed is continuously impaired due to a brain disease, such as a degenerative brain disease or a cerebrovascular disease.

Cognitive decline is also referred to as cognitive disorder, and refers to a state in which memory, attention, language ability, visuospatial ability, judgment, and the like are impaired. Cognitive decline corresponds to an intermediate stage between normal aging and dementia, and may progress to dementia. It is a comprehensive concept that encompasses everything from a decline in memory function accompanying the normal aging process to cognitive impairment corresponding to a pre-dementia stage. Conditions such as 'benign senescent forgetfulness,' 'age-associated memory impairment,' 'age-associated cognitive decline,' and 'cognitive impairment-no dementia (CIND)' may also be included within the scope of cognitive decline of the present invention.

In a further aspect, a food or medical use of the complex and/or a composition comprising the complex is provided herein. The complex and/or the composition comprising the complex is provided so as to be used as a food, a health functional food, a medicine, and the like.

In some embodiments, a method of treating or preventing a dementia and/or cognitive decline condition is provided, the method comprising applying or administering the complex of the present invention, or a composition comprising the complex, to a subject in need thereof. In a specific embodiment, such use provides, for example, an effect of treating or improving a symptom of dementia and/or cognitive decline in at least 5%, for example, 5, 10, 15, 20, 25, 30, 35, 40, 45, 50, 55, 60, 65, 70%, or more, of subjects administered the complex and/or the composition. For example, the improved effect may include not only an improvement in an individual ability, such as memory, attention, language ability, visuospatial ability, and judgment, but also an effect such as an improvement in sociability. In a specific embodiment, the use of the present invention is intended for adults aged 65 or older; however, the use is not particularly limited by age, and an adult suffering from early-onset dementia, vascular dementia, or the like, or an adult suffering from cognitive impairment at a young age, may also be included within the scope of use of the present invention.

Administration of the complex and/or the composition may be performed orally, sublingually, intravenously, intramuscularly, intradermally, transdermally, or subcutaneously, and may preferably be provided as oral administration.

In one embodiment, depending on the severity of the disease of the subject, the complex and/or the composition of the present invention may also be provided in an attachable formulation, such as a microneedle patch or a transdermal patch.

In one embodiment, a use of the complex of the pine needle extract and the galangal extract, and/or a composition comprising the complex, for a food or a medicine for preventing, improving or treating dementia and/or cognitive decline is provided. Preferably, the complex of the pine needle extract and the galangal extract is provided by mixing the galangal extract with the pine needle extract in a weight ratio of 1:1 to 1:8 (pine needle extract:galangal extract).

Preventing, improving or treating a dementia and/or cognitive decline condition relates, for example, to inhibiting full progression to such a condition in a subject at risk of dementia and cognitive decline. 'Treatment' refers to a therapeutic intervention that ameliorates a sign or symptom of a disease or a pathological condition after the sign or symptom has begun to develop. The term 'improvement' refers to a reduction in the severity of some or all clinical symptoms of a disease, a delayed onset of clinical symptoms, or a slower progression of a disease. 'Prevention' may be understood as reducing the risk of developing a pathological symptom in a subject who shows no signs of the disease or shows only early signs thereof.

The selection of a specific effective dose may be determined by one of ordinary skill in the art, taking into account a number of factors, including the disease to be treated or prevented, the associated symptoms, the medical history of the subject, the physical condition of the subject, such as the age, body weight, and/or immune status of the subject, the administered composition, and other factors known to one of ordinary skill in the art. The dose to be used in a formulation comprising the complex of the extracts of the present invention and/or a composition containing the complex may vary depending on the age, body weight, sex, mode of administration, health condition, and degree of disease of the subject, and may be administered in divided doses, once to several times a day, at predetermined time intervals, according to the judgment of a physician or a pharmacist. For example, the daily dose, based on the content of the active ingredient, may be 0.001 to 2 g/kg, preferably 0.5 to 500 mg/kg. The above-described dose is illustrative of an average case, and the dose may be higher or lower depending on individual differences.

In one embodiment, the subject in need thereof is a mammalian subject, for example, a human subject.

More specifically, it was confirmed that the complex and/or the composition according to the present invention has an anti-inflammatory activity of inhibiting the production of nitric oxide, an inflammatory mediator, in mouse-derived macrophage cell line RAW 264.7 cells in which an inflammatory response was induced with lipopolysaccharide (LPS), while simultaneously inhibiting the activity of acetylcholinesterase, a hydrolytic enzyme of the neurotransmitter acetylcholine.

Hereinafter, the present invention will be described in detail with reference to Examples and the like, in order to facilitate understanding of the present invention. However, the Examples according to the present invention may be modified into various other forms, and the scope of the present invention is not limited to the following Examples. The Examples of the present invention are provided to more fully explain the present invention to a person having an average level of skill known in the art. Unless otherwise specified, the blending ratio of each component is understood to be a weight ratio.

### Examples

### <Preparation Example 1> Preparation of Pine Needle Extract and Galangal Extract

### 1. Preparation of Pine Needle Extract

For the preparation of the pine needle extract, a dried raw material purchased through a domestic distributor was used. The dried pine needles were weighed, washed with water, and placed in an extractor at a weight ratio of raw material:solvent = 1:10, and extraction was performed at 60°C ± 10% for 6 hours; this process was repeated for a total of two extractions. 50% ethanol was used as the solvent. The pine needle extract was filtered and then concentrated under reduced pressure to obtain a concentrate of 30 brix (± 5 brix), which was then dried. The dried powder was homogenized to prepare the pine needle extract to be used in the present invention.

### 2. Preparation of Galangal Extract

For the preparation of the galangal extract, a dried raw material purchased through a domestic distributor was used. The dried galangal was weighed, washed with water, and placed in an extractor at a weight ratio of raw material:solvent = 1:10, and extraction was performed at 100°C ± 10% for 3 hours; this process was repeated for a total of two extractions. Water was used as the solvent. The galangal extract was filtered and then concentrated under reduced pressure to obtain a concentrate of 20 brix (± 5 brix), which was then dried. The dried powder was homogenized to prepare the galangal extract to be used in the present invention.

### 3. Preparation of Complex

The pine needle extract and the galangal extract prepared by the above-described process were mixed as follows and used as test substances (Table 1: Preparation of Complex).

**[Table 1]**

| **Classification** | **Pine Needle Extract (% by weight)** | **Galangal Extract (% by weight)** | **Sodium Phosphate Buffer (% by weight)** | **Complex Ratio (Pine Needle:Galangal)** |
|---|---|---|---|---|
| Example 1 | 88.9 | 11.1 | - | 8:1 |
| Example 2 | 80.0 | 20.0 | - | 4:1 |
| Example 3 | 66.7 | 33.3 | - | 2:1 |
| Example 4 | 50.0 | 50.0 | - | 1:1 |
| Example 5 | 33.3 | 66.7 | - | 1:2 |
| Example 6 | 20.0 | 80.0 | - | 1:4 |
| Example 7 | 11.1 | 88.9 | - | 1:8 |
| Comparative Example 1 | 11.1 | - | 88.9 | - |
| Comparative Example 2 | - | 88.9 | 11.1 | - |

### <Test Example 1> Confirmation of Component Content of Test Substances

### 1. Confirmation of Component Content of Pine Needle Extract

The pine needle extract prepared in Preparation Example was analyzed using high-performance liquid chromatography (HPLC) to determine the content of the major component of the pine needle extract.

More specifically, for preparation of a standard solution, an appropriate amount of the standard substance was dissolved in methanol to a concentration of 0.5 mg/mL to obtain a standard stock solution. The standard stock solution was dissolved by shaking and then diluted with methanol to obtain the standard solution. For preparation of a test solution, each extract was precisely weighed, methanol was added thereto, and the mixture was subjected to ultrasonic extraction. The resulting solution was filtered through a 0.22 to 0.45 µm PTFE or PVDF membrane filter to obtain the test solution.

As HPLC analysis conditions, a UV-visible spectrophotometric detector (measurement wavelength of 254 nm) was used as the detector, and the column was a stainless steel tube having an inner diameter of 4 to 6 mm and a length of 15 to 25 cm, packed with octadecylsilyl silica gel for liquid chromatography having a particle size of 5 to 10 µm. The column temperature was a constant temperature of approximately 30°C, and mobile phase A and mobile phase B were used as the mobile phase and controlled in a stepwise or gradient manner. Mobile phase A was set as a 0.1% aqueous phosphoric acid solution, and mobile phase B was set as methanol.

As a result, protocatechuic acid was identified as the major component in the pine needle extract, and the content of protocatechuic acid in the pine needle extract prepared according to the above-described Preparation Example was confirmed to be in the range of 0.05 to 0.35 mg/g (Table 2: Content of Protocatechuic Acid in Pine Needle Extract).

**[Table 2]**

| **Classification** | **Content of Protocatechuic Acid (mg/g)** |
|---|---|
| Measurement 1 | 0.05 |
| Measurement 2 | 0.35 |
| Measurement 3 | 0.26 |

### 2. Confirmation of Component Content of Galangal Extract

The galangal extract prepared in Preparation Example was analyzed using HPLC to determine the content of the major component of the galangal extract.

More specifically, for preparation of a standard solution, an appropriate amount of the standard substance was dissolved in 50% methanol to a concentration of 0.5 mg/mL to obtain a standard stock solution. The standard stock solution was dissolved by shaking and then diluted with 50% methanol to obtain the standard solution. For preparation of a test solution, each extract was precisely weighed, methanol was added thereto, and the mixture was subjected to ultrasonic extraction. The resulting solution was filtered through a 0.22 to 0.45 µm PTFE or PVDF membrane filter to obtain the test solution.

As HPLC analysis conditions, a UV-visible spectrophotometric detector (measurement wavelength of 254 nm) was used as the detector, and the column was a stainless steel tube having an inner diameter of 4 to 6 mm and a length of 15 to 25 cm, packed with octadecylsilyl silica gel for liquid chromatography having a particle size of 5 to 10 µm. The column temperature was a constant temperature of approximately 40°C, and mobile phase A and mobile phase B were used as the mobile phase and controlled in a stepwise or gradient manner. Mobile phase A was set as a 2% aqueous phosphoric acid solution, and mobile phase B was set as methanol.

As a result, 4-hydroxybenzaldehyde was identified as the major component in the pine needle extract, and the content of 4-hydroxybenzaldehyde in the pine needle extract prepared according to the above-described Preparation Example was confirmed to be in the range of 0.04 to 0.20 mg/g (Table 3: Content of 4-Hydroxybenzaldehyde in Galangal Extract).

**[Table 3]**

| **Classification** | **Content of 4-Hydroxybenzaldehyde (mg/g)** |
|---|---|
| Measurement 1 | 0.048 |
| Measurement 2 | 0.152 |
| Measurement 3 | 0.193 |

### <Test Example 2> Evaluation of Acetylcholinesterase Inhibitory Activity of Test Substances

To evaluate the efficacy of the test substances prepared in the Examples against cognitive decline, the inhibitory activity against acetylcholinesterase, a hydrolytic enzyme of the neurotransmitter acetylcholine, was evaluated by a modified Ellman's method.

More specifically, each test substance prepared in the Examples, tacrine as a positive control, and sodium phosphate buffer (pH 8.0) as a control were each dispensed in an amount of 10 µl into each well of a 96-well microplate, and then 120 µl of sodium phosphate buffer, 10 µl of 3 mM ATCI, and 30 µl of 10 mM DTNB reagent were sequentially dispensed into the wells, followed by a reaction at room temperature for 15 minutes. Thereafter, 20 µl of AChE enzyme was dispensed into the experimental group, and 20 µl of sodium phosphate buffer was dispensed into the blank, followed by a reaction for 30 minutes, and the absorbance was then measured at 412 nm using a microplate reader. The results were expressed as an inhibition rate relative to the control group, as shown in Equation 3 below, and turbidity was corrected (Equation 1) and color was corrected using the blank group (Equation 2), based on the absorbance measured at 880 nm. [Equation 1] $Turbidity correction = {A}_{412} - {A}_{880}$
A₄₁₂: absorbance at 412 nm after incubation
A₈₈₀: absorbance at 880 nm after incubation
[Equation 2] $Color correction = {A}_{test} - {A}_{blank}$
Aₜₑₛₜ: turbidity-adjusted absorbance of test well
A_{blank}: turbidity-adjusted absorbance of blank well
[Equation 3] $Inhibition rate \left(%\right) = \left\{1-\left(abs/abc\right)\right\} \times 100$
abs = turbidity and color-adjusted absorbance of test well after incubation
abc = turbidity and color-adjusted absorbance of control well after incubation

The expected efficacy of the complex was calculated using Colby's formula (Equation 4), and the synergistic effect thereof was shown in Table 4 by comparison with the measured value (Table 4: Acetylcholinesterase Inhibitory Activity of Test Substances). To calculate the predicted value of the acetylcholinesterase inhibitory activity of the complex according to Colby's formula, the acetylcholinesterase inhibitory activity of each of the pine needle extract and the galangal extract alone, according to the mixing ratio of each Example, was measured and applied to the calculation. [Equation 4] $E = X + Y - \left(X\times Y\right) / 100$
X: inhibition rate when Compound A is used at concentration a
Y: inhibition rate when Compound B is used at concentration b

**[Table 4]**

| **Classification** | **Predicted Value (%)** | **Measured Value (%)** | **Synergistic Effect Relative to Predicted Value (%)** |
|---|---|---|---|
| Example 1 | 91.22 | 80.60 | - |
| Example 2 | 75.27 | 78.02 | 3.65 |
| Example 3 | 51.08 | 66.49 | 30.17 |
| Example 4 | 32.03 | 35.41 | 10.55 |
| Example 5 | 32.86 | 43.01 | 30.89 |
| Example 6 | 38.42 | 56.45 | 46.93 |
| Example 7 | 47.03 | 72.05 | 53.20 |
| Comparative Example 1 | - | 31.75 | - |
| Comparative Example 2 | - | 22.39 | - |

As a result of the experiment, it was confirmed that, in all of the Examples except Example 1, the measured value was higher than the value predicted by Colby's formula, confirming a synergistic effect, and among these, a remarkable synergistic effect was confirmed in Example 3, Example 5, Example 6, and Example 7.

### <Test Example 3> Evaluation of Cytotoxicity of Test Substances

To evaluate the cytotoxicity of the test substances prepared in the Examples, cell viability was evaluated in SH-SY5Y, a human-derived neuronal cell line.

More specifically, SH-SY5Y cells being cultured in a separate culture plate were collected, seeded into a 96-well plate at a concentration of 2 x 10⁶ cells/well, cultured for 24 hours, treated with the test substance, and further allowed to react for 24 hours. Thereafter, the cells were treated with a WST reagent and cultured for 3 hours, and cell viability was evaluated by measuring the absorbance at 450 nm using a microplate reader, the results of which are shown in Table 5 (Table 5: Evaluation of Cytotoxicity of Test Substances).

**[Table 5]**

| **Classification** | **Concentration** | **Cell Viability (%)** |
|---|---|---|
| Pine Needle Extract | 12 µg/ml | 93.17±9.51 |
| Pine Needle Extract | 25 µg/ml | 84.97±9.64 |
| Pine Needle Extract | 50 µg/ml | 77.90±7.92 |
| Galangal Extract | 25 µg/ml | 102.81±5.24 |
| Galangal Extract | 50 µg/ml | 98.10±1.05 |
| Galangal Extract | 100 µg/ml | 95.29±3.81 |

As a result of the experiment, it was confirmed that the galangal extract showed almost no cytotoxicity, even at a concentration of 100 µg/ml, whereas the pine needle extract showed concentration-dependent cytotoxicity. In addition, it was confirmed that, in the complex of the present invention, when the galangal extract accounted for at least half of the weight of the complex, the complex showed almost no cytotoxicity even at a concentration of 100 µg/ml, and accordingly, test substances having the compositions of Example 4 to Example 7 of Table 1 were used in the subsequent cell test.

### <Test Example 4> Evaluation of Anti-Inflammatory Effect of Test Substances

To evaluate the anti-inflammatory effect of the test substances prepared in the Examples, the inhibitory effect on the production of nitric oxide, an inflammatory mediator, was evaluated in mouse-derived macrophage cell line RAW 264.7 cells in which an inflammatory response was induced with lipopolysaccharide (LPS).

More specifically, RAW 264.7 cells being cultured in a separate culture plate were collected, seeded into a 96-well plate at a concentration of 5 x 10⁴ cells/well, cultured for 24 hours, treated with the test substance at a concentration of 100 µg/ml, and further allowed to react for 24 hours. Thereafter, the cells were treated with lipopolysaccharide at a concentration of 10 ng/ml and stimulated for 24 hours, and the supernatant was treated with Griess reagent and allowed to react for 30 minutes, after which the production of nitric oxide in the cell supernatant was evaluated by measuring the absorbance at 540 nm using a microplate reader. The results were expressed as an inhibition rate relative to the stress group in which an inflammatory response was induced with LPS, as shown below (Table 6: Nitric Oxide Production-Inhibitory Activity of Test Substances). [Equation 5] $Inhibition rate \left(%\right) = \left\{1-\left(abt/abs\right)\right\} \times 100$
abt = absorbance of test well after incubation
abs = absorbance of stress well after incubation

**[Table 6]**

| **Classification** | **NO Production-Inhibitory Activity (%)** |
|---|---|
| Example 4 | 4.58±0.90 |
| Example 5 | 5.01±0.58 |
| Example 6 | 13.71±0.91 |
| Example 7 | 20.03±2.45 |

As a result of the experiment, the nitric oxide production-inhibitory activity was confirmed in all of the Examples, and excellent NO production-inhibitory activity was confirmed in Example 6 and Example 7.

### <Test Example 4-1> Evaluation of Concentration-Dependent Anti-Inflammatory Effect of Test Substance

To evaluate the concentration-dependent anti-inflammatory effect of Example 7, which was confirmed in Test Example 2 to have the highest synergistic effect, the inhibitory effect on the production of nitric oxide, an inflammatory mediator, was evaluated in mouse-derived macrophage cell line RAW 264.7 cells in which an inflammatory response was induced with LPS.

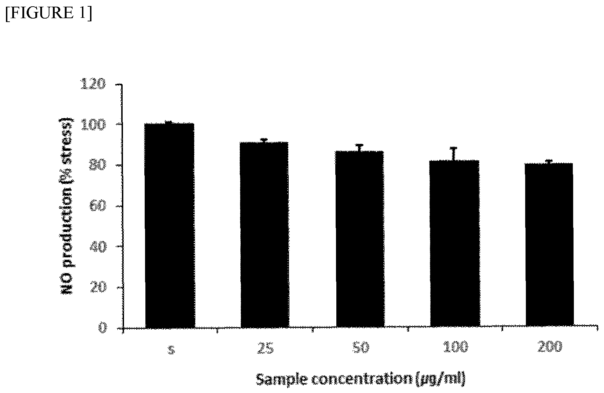

More specifically, RAW 264.7 cells being cultured in a separate culture plate were collected, seeded into a 96-well plate at a concentration of 5 x 10⁴ cells/well, cultured for 24 hours, treated with the test substance at different concentrations, and further allowed to react for 24 hours. Thereafter, the cells were treated with lipopolysaccharide at a concentration of 10 ng/ml and stimulated for 24 hours, and the supernatant was treated with Griess reagent and allowed to react for 30 minutes, after which the production of nitric oxide in the cell supernatant was evaluated by measuring the absorbance at 540 nm using a microplate reader. The results were expressed as an inhibition rate relative to the stress group in which an inflammatory response was induced with LPS, as shown below (FIG. 1). [Equation 6] $Inhibition rate \left(%\right) = \left\{1-\left(abt/abs\right)\right\} \times 100$
abt = absorbance of test well after incubation
abs = absorbance of stress well after incubation

As shown in FIG. 1, when the complex of the pine needle extract and the galangal extract of Example 7 was treated, the production of nitric oxide was inhibited in a concentration-dependent manner, confirming that the test substance has excellent anti-inflammatory activity.

### <Test Example 5> Evaluation of Cognitive Function-Improving Effect of Test Substance - Y-Maze Test

To evaluate the cognitive function-improving effect of the test substance prepared in the Examples, cognitive function was evaluated through a Y-maze test, using mice in which an Alzheimer's disease-like state was induced by intracerebroventricular injection of beta-amyloid.

### [Experimental Animals]

A total of 48 male mice (C57BL/6N), 8 weeks old and weighing 20 ± 2.2 g, were divided into 6 groups of 8 mice each, namely a normal group, an induction group, a pine needle extract:galangal extract 1:1-administered group (Example 4), a pine needle extract:galangal extract 1:4-administered group (Example 6), a pine needle extract:galangal extract 1:8-administered group (Example 7), and a positive control group. Beta-amyloid was administered to the remaining 5 groups, other than the normal group, to induce an Alzheimer's disease-like state.

### [Administration of Beta-Amyloid]

Beta-amyloid was dissolved in sterilized 0.1 M phosphate-buffered saline (pH 7.4) and prepared at a concentration of 1 µg/µl; to induce aggregation, the solution was stored at 37°C for 1 week prior to administration, and then administered into the third ventricle of the brain. The experimental animals were anesthetized with Avertin, and then 5 µl of the aggregated beta-amyloid solution was intracerebrally administered at a rate of 2 µl/min using a stereotaxic apparatus.

### [Administration of Test Substance]

For 3 weeks from the day of beta-amyloid injection, the test substance was orally administered by direct intragastric administration using a syringe equipped with a sonde. For smooth administration, 0.5% carboxymethyl cellulose was used as an excipient, and in the normal group and the induction group, only the excipient, without the test substance, was administered (Table 7: Test Substance and Dose for Each Test Group).

**[Table 7]**

| **Test Group** | **Administered Substance** | **Dose (mg/kg/day)** | **Administration Volume (ml/kg/day)** |
|---|---|---|---|
| Normal group (NC) | Excipient | - | 10 |
| Induction group (AB) | Excipient | - | 10 |
| AB induction + 1:1-administered group | Pine needle:galangal 1:1 (Example 4) | 100 | 10 |
| AB induction + 1:4-administered group | Pine needle:galangal 1:4 (Example 6) | 100 | 10 |
| AB induction + 1:8-administered group | Pine needle:galangal 1:8 (Example 7) | 100 | 10 |
| AB induction + positive control group | Morin | 10 | 10 |

[Y-Maze Test] A Y-maze test was performed to evaluate the spatial cognitive ability of the experimental animals. The Y-maze is composed of three identical arms (A, B, and C; each 6 cm wide, 30 cm long, and 20 cm high) in the shape of a 'Y', each with a closed end. Each experimental animal was carefully placed at the end of one arm, allowed an acclimation period of 2 minutes, and then allowed to freely explore the Y-shaped arms for 8 minutes. At this time, a case in which the body of the animal entered a given arm beyond an initial distance of 10 cm was recognized as entry into that arm, and the number and sequence of entries into each arm were measured using a camera installed on the ceiling, and the spontaneous alternation behavior was evaluated using a video tracking device. Spontaneous alternation behavior was defined as sequential entry into all three arms, that is, ABC, BCA, CAB, and the like, and accordingly, the percent spontaneous alternation behavior was calculated according to the following formula.$\begin{matrix}% spontaneous alternation \\ = actual number of alternation / \left(total number of arm entries-2\right) \times 100\end{matrix}$ Equation 7

As a result of the experiment, it was confirmed that there was no significant difference in the number of entries into the maze among the normal group, the induction group, the test substance-administered groups, and the positive control group. However, it was confirmed that the induction group showed a marked decrease in spontaneous alternation (%) as compared with the normal group, whereas the group administered the pine needle:galangal 1:8 complex (Example 7) showed a result exceeding that of the normal group, as compared with the induction group, confirming a remarkable and significant cognitive function-improving effect (a 39.21% improvement relative to the induction group). The group administered the pine needle:galangal 1:1 complex (Example 4) and the group administered the pine needle:galangal 1:4 complex (Example 6) showed an excellent cognitive function-improving effect, with the spontaneous alternation improved by 16.73% and 11.59%, respectively, relative to the induction group (Table 8: Spontaneous Alternation, FIG. 2).

**[Table 8]**

| **Test Group** | **Spontaneous Alternation (%)** | **Effect (Relative to Induction Group)** |
|---|---|---|
| Normal group (NC) | 65.55 ± 4.81 | - |
| Induction group (AB) | 50.39 ± 12.66 | - |
| Induction+1:1-administered group (Example 4) | 58.82 ± 11.13 | 16.73% improvement |
| Induction+1:4-administered group | 56.23 ± 5.48 | 11.59% improvement |
| (Example 6) | | |
| Induction+1:8-administered group (Example 7) | 70.15 ± 11.72 | 39.21% improvement |
| Induction+Morin | 70.68 ± 9.32 | - |

### INDUSTRIAL APPLICABILITY

The complex and/or the composition of the present invention has an effect of treating, preventing, or improving dementia, and provides an effect of preventing, improving, or treating cognitive decline, and therefore can be provided as a health functional food or a pharmaceutical product.

## Claims

1. A composition for preventing, improving, or treating dementia and/or cognitive decline, comprising a complex of pine needle extract and galangal extract as an active ingredient.

2. The composition of claim 1, wherein the pine needle extract is obtained by extracting pine needles with water, a lower alcohol having 1 to 4 carbon atoms, or a mixed solvent thereof, followed by concentration.

3. The composition of claim 1, wherein the galangal extract is obtained by extracting galangal with water, a lower alcohol having 1 to 4 carbon atoms, or a mixed solvent thereof, followed by concentration.

4. The composition of claim 1, wherein the pine needle extract or the galangal extract is a concentrate or a dry powder.

5. The composition of claim 1, wherein the complex of the pine needle extract and the galangal extract is obtained by mixing the galangal extract with the pine needle extract in a weight ratio of 1:1 to 1:8 (pine needle extract:galangal extract).

6. The composition of claim 1, wherein the complex of the pine needle extract and the galangal extract is **characterized by** an anti-inflammatory action, acetylcholinesterase inhibition, and improvement of beta-amyloid-induced cognitive decline.

7. The composition of claim 1, wherein the pine needle extract contains 0.05 to 0.35 mg/g of protocatechuic acid.

8. The composition of claim 1, wherein the galangal extract contains 0.04 to 0.20 mg/g of 4-hydroxybenzaldehyde.

9. The composition of any one of claims 1 to 8, wherein the composition is a health functional food composition for preventing or improving dementia and/or cognitive decline.

10. A health functional food product comprising the composition of claim 9.

11. The composition of any one of claims 1 to 8, wherein the composition is a pharmaceutical composition.

12. A pharmaceutical product comprising the composition of claim 11.

13. A method of improving or treating dementia and/or cognitive decline in a subject, comprising administering an effective amount of a complex of pine needle extract and galangal extract to a subject in need thereof.

14. The method of claim 13, wherein the complex of the pine needle extract and the galangal extract is obtained by mixing the galangal extract with the pine needle extract in a weight ratio of 1:1 to 1:8 (pine needle extract:galangal extract).

15. Use of a complex of pine needle extract and galangal extract for a food or a medicine for preventing, improving or treating dementia and/or cognitive decline.

16. The use of claim 15, wherein the complex of the pine needle extract and the galangal extract is obtained by mixing the galangal extract with the pine needle extract in a weight ratio of 1:1 to 1:8 (pine needle extract:galangal extract).
